(19) **Europäisches Patentamt / European Patent Office / Office européen des brevets**

(11) **EP 3 531 896 B1**

(12) **EUROPEAN PATENT SPECIFICATION**

(45) Date of publication and mention of the grant of the patent:
**05.10.2022 Bulletin 2022/40**

(21) Application number: **17798128.9**

(22) Date of filing: **26.10.2017**

(51) International Patent Classification (IPC):
**A61B 5/00** *(2006.01)* **G10H 1/00** *(2006.01)*

(52) Cooperative Patent Classification (CPC):
**G10H 1/0025; A61B 5/441; A61B 5/448; A61B 5/7415; A61B 5/7455;** A61B 5/1077; A61B 5/6898; A61B 5/7257; G10H 2220/371

(86) International application number:
**PCT/EP2017/077515**

(87) International publication number:
**WO 2018/078052 (03.05.2018 Gazette 2018/18)**

(54) **A METHOD FOR INFORMING A USER ABOUT A CONDITION OF HUMAN KERATINOUS**

VERFAHREN ZUR BENACHRICHTIGUNG EINES BENUTZERS ÜBER EINEN ZUSTAND VON MENSCHLICHEM KERATIN

PROCÉDÉ POUR INFORMER UN UTILISATEUR À PROPOS D'UN L'ÉTAT DE KÉRATINE HUMAINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2016 JP 2016211669**

(43) Date of publication of application:
**04.09.2019 Bulletin 2019/36**

(73) Proprietors:
• **L'Oreal**
  **75008 Paris (FR)**
• **EL Produce Inc.**
  **Tokyo 156-0051 (JP)**

(72) Inventors:
• **NOMURA, Mariko**
  **Kawasaki**
  **Kanagawa 213-0012 (JP)**
• **KAMIGORI, Akiko**
  **Kawasaki**
  **Kanagawa 213-0012 (JP)**
• **VELLEMAN, Damien**
  **Kawasaki**
  **Kanagawa 213-0012 (JP)**

• **FLAMENT, Frédéric**
  **92110 Clichy (FR)**
• **IDE, Hiroaki**
  **Tokyo 156-0051 (JP)**
• **ISSHIKI, Konomi**
  **Tokyo 156-0051 (JP)**
• **TANAKA, Fumihisa**
  **Tokyo 156-0051 (JP)**
• **URAGAMI, Sakie**
  **Tokyo 156-0051 (JP)**

(74) Representative: **Nony**
  **11 rue Saint-Georges**
  **75009 Paris (FR)**

(56) References cited:
| | |
|---|---|
| EP-A1- 1 691 195 | EP-A1- 1 791 111 |
| EP-A2- 0 158 336 | EP-A2- 1 130 570 |
| WO-A1-2016/153132 | JP-A- 2004 159 830 |
| JP-A- 2006 158 526 | JP-B2- 2 904 616 |
| JP-B2- 2 941 114 | US-A1- 2004 171 962 |
| US-A1- 2012 041 282 | |

**Description**

## FIELD OF THE INVENTION

The present invention relates to methods and devices for evaluating human keratinous hair material, and for informing a user about a condition of said material.

## BACKGROUND OF THE INVENTION

Physical characterization of keratinic substrates has become with years more and more accurate and complex to analyse in its plurality (ethnics, environment ...) and to demonstrate sincere and global performance of regimen and therefore support claims. Very often combination of several dimensions is mandatory to conclude about impact on full-face skin or hairs in rigorous and holistic way.

The results afforded by all these techniques (coupled to scientific expertise) in the assessment of the effect of topically applied products, constitute in most cases parts of necessary legal claim substantiation, i.e. an official dossier that often regulates the right to market. However, they remain highly difficult to communicate to a large public (cosmetic consumers or dermatological patients) due to their increasing complexity coupled to a poor- or low- scientific knowledge of most people.

Application US 2015/0201889 describes the uses of sonification to interpret brain scans. Sound serves as a complementary tool to review, analyze, and diagnose.

Application JP 2012-226472 describes a method and device to identify a feature of an object by sound by mainly using a signal processing approach.

US 8,440,902 relates to a method for representing multidimensional numerical data via a plurality of variable timbre channels into audio-frequency waveform. This audio-frequency waveform is perceived by a user as an audio tone.

US 2012/041282 A1 (NICHOL JAMIE GORDON [US] ET AL) 16 February 2012 (2012-02-16) discloses measuring Skin/Scalp/Hair parameters and sending them through an audio modulated stream (e.g. DTMF) to a user device.

EP 1 691 195 A1 (KAO CORP [JP]) 16 August 2006 (2006-08-16) relates to the provision of an audio frequency reflecting hair quality using a vibration analysis through a microphone sensor coupled to a comb like measurement device and for assessing the evenness/damage scale of the hair.

JP 2004 159830 A (LION CORP) 10 June 2004 (2004-06-10) relates to determining the hair structure by analysing the vibration detected by comb-like measurement device friction. A friction sound generated by the sensor is amplified and provided to the user for evaluation. Frequency analysis is carried out on measured vibrations detected by the sensor.

JP 2 941114 B2 (POLA CHEM IND INC) 25 August 1999 (1999-08-25) discloses using optical means to translate hair condition parameters into musical notes.

JP 2 904616 B2 (POLA CHEM IND INC) 14 June 1999 (1999-06-14) discusses using a laser and optical sensor to translate skin condition parameters such as roughness into musical notes.

There thus exists a need to further improve and facilitate perception of users of the result of an evaluation of a skin or hair or other human keratinous materials condition.

## SUMMARY OF THE INVENTION

According to a first aspect, the present invention relates to a method according to appended claim 1.

Thanks to the present invention, a sensory signal easy to be perceived by the user is generated.

This allows transforming and enhancing a consumer experience in the dermatological and cosmetic fields. This facilitates communicating to the user an indication of a performance or a diagnostic result and increases personalization and coaching potentialities.

Be able to transform in a chosen dynamic sensory way that could be delivered in all digital support like smartphones (succession of sounds, image in motion, tactile vibrations...) every kind of objective assessments or physical data (for example skin wrinkles or pores roughness, hair surface damages, skin and hair shininess, skin and hair colour heterogeneities, skin and scalp inflammation, skin or hair structure alterations, skin water content, hair porosity...) is a pathway to individualization of consumer experience by making her/him understand and feel a diagnosis or an efficiency of a cosmetic treatment.

The shaping of measurement data may comprise filtering of the measurement data, preferably with a low-pass or a high-pass filter. Any other filtering techniques may be used in order to generate from the measurement data shaped data appropriate for user signal generation. For example, high or low pass filter, band stop or pass filter, offset and so on.

Depending on the measuring device that is used, the data generated by the measuring device may be already shaped data or not.

The shaping may also comprise any desired transformation useful for user signal generation, for example a transformation of a spatial to a time domain. This may prove especially useful when the user sound signal is generated directly in real time based on shaped data.

According to the present invention, shaping comprises generating data based on a comparison of the measurement data with reference data; for example the shaped data takes various predefined formats or contents based on a comparison with reference data. The reference data relates to various degree of damages to hair surface.

The shaping may comprise generating data based on an adaptation of filtered measurement data to a device used for generating the user sound or tactile signal, for example, a haptic device. For example, the filtered measurement data is in a form of vibration curves. These curves are scaled and /or offset to fit the haptic device. The user tactile signal is generated based on this adaptation.

In an alternative example departing from the claimed invention, the measurement data may be generated by a measuring device that is configured for measuring a skin condition.

Such a skin condition may be, inter alia, selected among skin roughness, a degree of a skin wrinkles or pore roughness, skin shininess, skin color heterogeneities, skin or scalp inflammation, skin structure alteration, skin water content, skin color, trans epidermal water loss, skin hydration, skin pigmentation, skin elastic properties, and skin structure.

The measuring device is configured to generate measurement data relating to a hair condition.

Such a hair condition comprises hair coefficient of friction, and may further be selected among hair surface damages, hair shininess, hair color heterogeneities, hair structure alteration, hair porosity, hair structure.

A further example departing from the present invention is not restricted to skin or hair evaluation and may also apply to other human keratinous materials such as lips or nails.

Preferably, the user signal is a sound signal comprising a sound arrangement, for example sound generation, that is generated based on a musical conversion.

In other words, the user signal is preferably a sound arrangement that makes it easy for the user to distinguish between different conditions of the keratinous materials under evaluation.

The method may comprise generating a user signal that comprises different sound arrangements depending on respective degrees of the condition of the keratinous materials under evaluation. The user signal is generated in real time, at a same time as the measuring device is operated.

For example, the condition is a damage level to hair and the sound arrangement comprises sounds of different pitch, amplitude, envelope and/or timbre depending on the damage level.

A variation of pitch, amplitude, envelope and/or timbre may thus be generated based on the result of a comparison of a shaped data with reference data.

The user signal may comprise musical notes and preferably a melody.

In a variant, the user sound or a tactile signal is generated by a signal conversion, for example from the spatial to time and frequency domains; this allows to easily generate sounds that depend on a profile of the data in the spatial domain.

The method may comprise generating a spectrogram based on the shaped data, for example the sound generated from the shaped data, and may comprise generating a sound and/or tactile user signal based on the spectrogram.

The user sound or tactile signal may be generated based on a customized user profile. The method may comprise an acquisition of a user preference, for example a musical instrument, and the sound user signal may be generated based on this user preference, for example music notes played with this instrument.

A further object of the present invention is a device according to appended claim 8, adapted to perform the method according to appended claim 1.

The acquisition means may comprise a device configured to be moved along a portion of the human keratinous materials, for example skin or hair, and to generate measurement data depending of an interaction between the measuring device and the portion under evaluation.

The measuring device is sensitive to friction and provides an information representative of a coefficient of friction of hair.

The measuring device may comprise a sensor that captures pressure modifications during rubbing of the measuring device along skin or hair. Fluctuations of these pressure modifications may be transformed into a user signal such as music via several mathematical transformations.

In an alternative to the present invention, the measuring device may be selected among colorimeters, evaporimeters, impedance or capacitive measurement sensors, intensity measurement devices, OCT (Optical Coherence Tomography) and ultrasound devices.

The processing means may be any electronic circuit that is capable of performing the required operations.

The processing means may comprise an integrated circuit, for example a microprocessor or microcontroller, that may be part of the measuring device or of a user device that is coupled to the measuring device and that receives data from the latter.

For example, the processing means is a personal computer to which the measuring device is connected via any connection means, such as a wired connection or wireless connection, for example Bluetooth, WiFi or LiFi.

The processing means may also comprise a mobile phone to which the measuring device is connected.

The generating means may be a separate device from the processing means or may be the same device as the processing means, when the processing means already possesses sound and/or tactile capabilities.

For example, the processing means and the generating means are constituted by a same mobile phone that is coupled to the measuring device.

The generating means may also be a computer equipped with an audio processing card and speakers.

When the user signal is a tactile signal, the signal may be generated by a mobile phone equipped with a haptic screen, by using the vibration capabilities of the latter.

The tactile signal may be any tactile signal generated by an appropriate interface that is configured to generate a tactile pattern using piezoelectric elements, such as electro vibration electro attraction, or electro lubrication.

A further object of the present invention is a method for evaluation of a cosmetic effect of a cosmetic treatment, comprising:

a) performing an evaluation of condition of human keratinous materials with the method of the invention as defined above;
b) performing the cosmetic treatment; and
c) performing an evaluation of the same condition with the method of the invention as defined above.

At steps a) and c), the user may receive, preferably in real time, a sound or a tactile signal that is representative of the condition of zone under evaluation.

The user may perceive a difference within the user signals that were generated and any advice or explanation may be provided either automatically or by a third person to him/her; an indication may be provided to the user about the effect of the treatment.

The cosmetic treatment may comprise application of a cosmetic product to at least one of skin, hair, nails and/or lips.

The cosmetic treatment may also comprise cleaning the keratinous materials.

The cosmetic treatment may be performed using any device that is different from the measuring device. In a variant, the device that is used for performing the cosmetic treatment also has measuring capabilities and is capable of generating the measurement data.

**BRIEF DESCRIPTION OF THE FIGURES**

Further advantages and characteristics of the present invention may appear in relation to the following description of non-limited embodiments of the present invention and in view of the accompanying drawings in which:

- Figure 1 is a block chart of an exemplary method according to the present invention;
- Figure 2 corresponds to raw measurement data;
- Figure 3 corresponds to shaped data after filtering of the raw data of Figure 2;
- Figure 4 illustrates conversion from coefficient of friction data to music note resolution;
- Figure 5 illustrates a sound arrangement of data;
- Figure 6 illustrates a further arrangement of data to generate a user sound signal;
- Figure 7 illustrates expressing frictional data as pure tone;
- Figures 8A and 8B represent spatial profiles of examples of measurement data;
- Figures 9A and 9B correspond to profiles in time domain after mathematical transformation of the data of Figures 8A and 8B;
- Figures 10A and 10B illustrate a further transformation of data of Figures 9A and 9B;
- Figure 11A and 11B illustrate a final transform of data of Figures 10A and 10B to generate acoustic signals that could be perceived by human beings; and
- Figure 12 is a flow chart of an example of transformation of measurement data into tactile user signal.

**DETAILED DESCRIPTION**

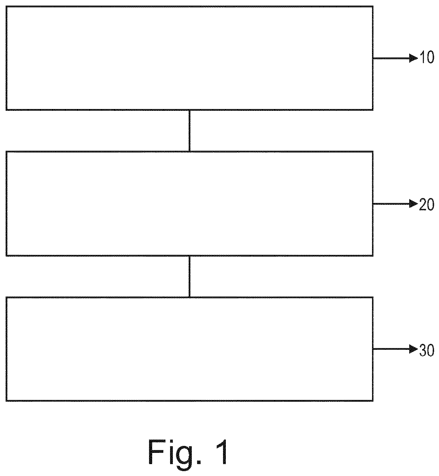

The method according to the present invention comprises as illustrated in Figure 1 a step 10 of obtaining via a measuring device measurement data about a zone of keratinous hair material.

The measuring device is for example configured for being rubbed against the hair and is for example available under a highly sensitive haptic probe (HRT, Company Trinity Lab. Inc. Tokyo, Japan)

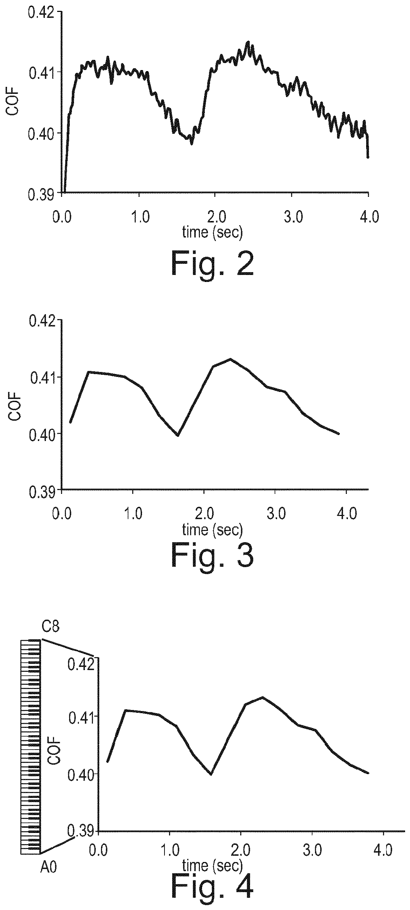

Such a device generates raw measurement data as illustrated in Figure 2 when the device is rubbed along a hair shaft.

The method according to the present invention comprises a further step 20 of shaping the measurement data into appropriate shaped data that are capable of being further processed to generate at step 30 a user sound and/or tactile signal.

In the example of raw data of Figure 2, the shaping 20 may comprise a high pass filter to filter out the noise. The raw data sampled at a rate of every 10ms is for example taken peak points at a rate of every 125ms. Thanks to the high pass filter, values between each two adjacent points are smoothed with moving average and a filtered data is obtained as shown in Figure 3.

The generating step 30 may comprise as illustrated in Figure 4, a first conversion from the filtered data to music scale resolution. This conversion may consist in one example in mapping 88 keys between maximum and minimum values of friction which are identified for a hair fiber. The maximum and minimum values of friction are identified based on friction values for a highly damaged hair and a natural hair following a hair treatment.

A sound may be generated at this step, consisting of a mapped note being hit every 125ms. Accordingly, every peak of data corresponds to one of the 88 keys.

Preferably, the music note conversion is carried out so as to avoid a too high pitch that may generate a too wide music range that would be difficult to listening.

The conversion may avoid generating a tone that is too low and difficult to listening or provide bad image to the user. At the same time, the conversion is done as to avoid a noisy sound and a bad feeling of the user.

The generating step 30 may further comprise, after narrowing the music note range, a selection of an instrument and of a volume.

The selection of the instrument may be predefined depending for example of the music range. Preferably, the instrument is selected based on a customized user profile. The method may thus comprise inputting user preferences before the sound signal is generated. The user preferences may relate to a preferred instrument.

The sound may also be generated based on a brand feature. For example, if a cosmetic product advertisement uses melodies of a predefined instrument, and the evaluation is carried out at the point of sale of products of this brand, the sound user signal may be generated by using the same instrument as for the advertisement.

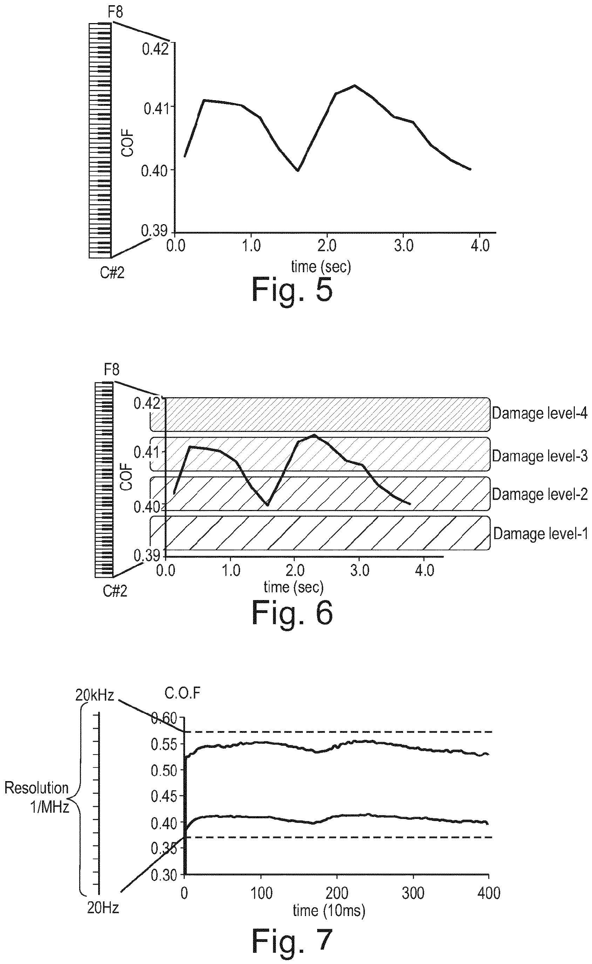

The method may further comprise, as illustrated in Figure 5, selection of a set of note within 88 initially mapped notes. The basis of the selection is to obtain a specific melody for each substrate and measurement method.

For example, figure 5 is relating to hair friction. The range of each note is fixed proportionally between total amount of selected notes and minimum and maximum potential values:

$$Range\ of\ Note = \frac{Maximum\ Friction\ Value - Minimum\ Friction\ Value}{Total\ Number\ of\ Notes}$$

When a friction value is within the range of a note, the corresponding note is played.

For example, a note is played at each 125ms, corresponding to the peak value and/or value of moving average etc, from a sound database containing prerecorded music note played by different music instruments. Background music can be added to the final sound and its volume is adjustable.

In the example of Figure 6, the damage of the hair is mapped into four categories corresponding each to a respective damage level and the sound is generated based on the category that corresponds to the user.

Accordingly, on Figure 6, as the shaped data evolves between extreme values that are within the lower boundary of damage level 2 and the upper boundary of damage level 3, the user will hear a sound that corresponds to predefined sounds for damage level 2 and damage level 3.

Through music note conversion is a preferred way to inform a user about a condition of his hair, it is possible without going beyond the scope of the invention to generate a user sound signal without music note conversion.

For example, as illustrated in Figure 7, a value for a coefficient of friction of hair is converted into a sound, and the frequency of the sound is shown as a spectrogram by FFT to see the frequency features.

In the example of Figure 7, one understands that the sound that is generated is different for the two measurement data corresponding to coefficient of friction values in the order of 0.4 for the lower curve and in the order of 0.5 for the upper curve.

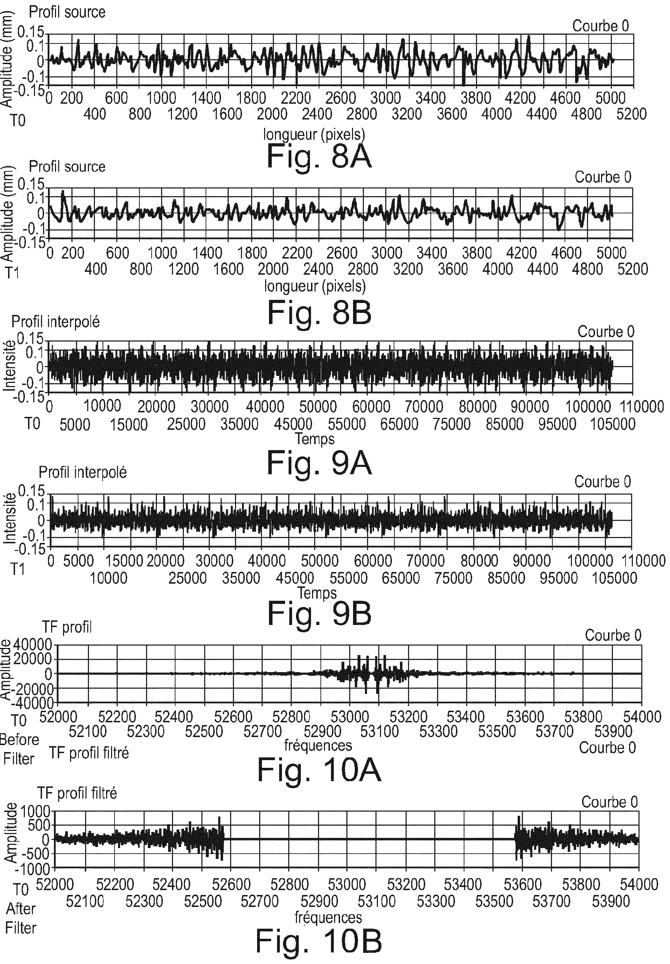

Another example of a mathematic transformation will be described in reference to Figures 8A to 10 B.

Figures 8A and 8B show a profile obtained with a measuring device that is rubbed against the skin for measuring rugosity of skin surface. The profile is a real roughness profile which is extracted from projection images of fringes acquired on fingerprints. A skin area of about 18×14mm is measured and profiles are obtained by 3D acquisition with 14μm resolution.

Five parallel profiles of 1000 pixels are plotted and each profile has a length of 14 mm. The five profiles are concatenated to form a 70mm profile.

Figure 8A corresponds to a measurement data at a first measurement time T0 and Figure 8B at a second measurement time T1.

Figures 9A and 9B correspond to profiles after a mathematical transformation of the spatial profile of Figures 8A and 8B into time profiles. The first part of the mathematical transformation is using interpolation to add a sufficient number of points. The profile remains the same, but a high sampling is obtained. The second part of the mathematical transformation is related to profile duplication. The profile duplication creates artificial frequencies, but thanks to a "band stop filter" as explained hereunder, this artificial frequencies problem is corrected. In the end of the interpolation and duplication, a signal sampled at 22 KHz which has a significant duration is obtained (here about 10 seconds).

Figures 10A and 10B correspond to data of Figures 9A and 9B after a further mathematical transformation by Fourier transform. After the Fourier transform, a small amplification may be applied to improve the signal strength. A filtering may be applied to remove the artificial frequencies introduced when profile duplication was carried out. The objective of this further mathematical transformation is to isolate parts of a spectrum which are most influenced by the measurement. The filtering may be a band-stop filtering.

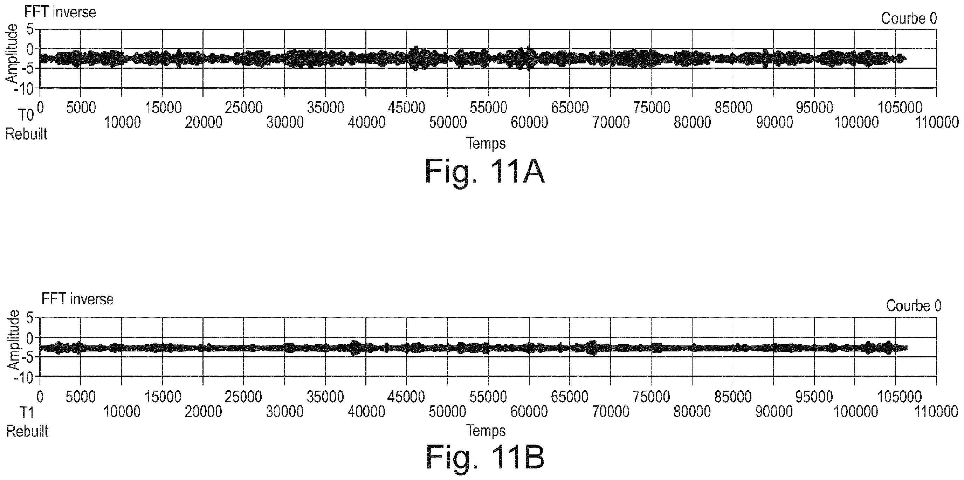

Figures 11A and 11B correspond to the frequency content of Figures 10A and 10B after a mathematical transformation into an audible signal in the range 20-40 Hz to 20 KHz. The mathematical transformation here is an inverse Fourier Transform.

Figure 11A and 11B show a type of signal obtained after the Inverse Fourier Transform. An audio file in ".wav" format is exported after obtaining a modified signal from the inverse Fourier Transform.

The present invention is not limited to a user signal that is a sound signal. The user signal may be a tactile signal.

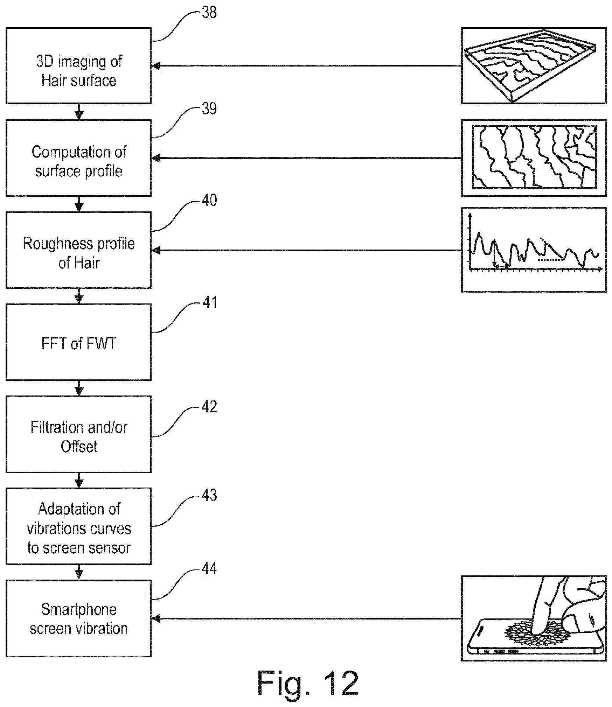

Figure 12 illustrates a method for generating a tactile user signal. The initial measurement in this example relates to hair surface. A roughness profile of hair may be generated at step 40 based on prior 3D imaging of hair surface at step 38 and computation of surface profile at step 39. Vibration curves 43 for a haptic interface may be generated by a mathematical transform such as FFT or FWT at step 41 and filtration and/or application of offset at step 42. These curves may be further adapted if appropriate to allow generation of the desired tactile pattern on the haptic interface. The latter may be part of a smart phone tactile screen.

The present invention is not limited to the disclosed embodiments.

In particular, the present invention applies to a wild variety of measuring devices and conditions of human keratinous hair material

In an example departing from the present invention, the present disclosure can be applied to a colorimeter and a condition of color of the skin. The color of the skin is an important feature to reveal sun exposure and classical bleaching.

An electronic interface allows to extract from a colorimeter $L^*$ and $a^*$ values (the higher the $L^*$ the paler the skin, the higher the $a^*$ the redder the skin) of a studied skin site and these values are further converted into sound by a mathematical transformation. Thresholds of changes in $L^*$ and $a^*$ (0 to 1, 0 to 2 or 3 units or above) values may be fixed at convenience, and changes in sound frequency can therefore be detected and heard as possible warnings (too intense sun exposure) to the users.

Similarly, the classical bleaching side effect of topically applied corticoids in dermatology, or a cosmetic whitening action can also be transformed into sounds.

In addition, the present example can also be used for sun protection. A UV sensor coupled with the method of present invention can help generate a user sound signal to warn sun-exposed subjects about their skin health status. This UV sensor can be further coupled with connected instruments such as mobile phone for generating the user sound signal.

In a further example, the present disclosure can be further applied to an evaporimeter and a condition of skin Trans Epidermal Water Loss (TEWL). The insensitive water flux that crosses the outermost layer of the skin (in g/m2/hour) can be indicated with sound.

The disclosure can be further applied to

- an impedance measurement sensor and a condition of skin hydration;
- an intensity measurement device and a condition of skin pigmentation;
- a biophysical recording device and a condition of skin elastic properties;
- a global state of an area measurement device (face, lips, hairs) and a condition of shininess.

**Claims**

1. A method for informing a user about a condition of human hair, comprising:

   a) obtaining via a measuring device measurement data about a zone under evaluation, wherein the measuring device is configured to generate measurement data relating to the hair condition, the measuring device being sensitive to friction and providing a coefficient of friction of hair;

b) shaping the measurement data to obtain shaped data, the shaping comprising generating data based on a comparison of the measurement data with reference data relating to various degrees of damages to hair surface; and **characterised by**

c) generating, in real time, based on the shaped data a sound user signal and/or tactile user signal to be perceived by the user, a frequency of the user signal depending on said shaped data.

2. The method of claim 1, wherein the shaping comprises filtering of the measurement data.

3. The method of claim 1 or 2, wherein the user signal comprises musical notes.

4. The method of claim 3, wherein the user signal comprises a melody.

5. The method of claim 3 and/or 4, wherein a variation of pitch, amplitude, envelope, and timbre is generated based on the result of a comparison of the shaped data with the reference data.

6. The method of claim 1 or 2, the method comprising generating a spectrogram based on the shaped data and generating a sound and/or tactile user signal based on the spectrogram.

7. The method of any one of claims 1 to 6 wherein the user sound or tactile signal is generated based on a customized user profile.

8. A device configured to perform the method of any one of claims 1 to 7, the device comprising:

- acquisition means for generating measurement data about human hair;
- processing means for processing the measurement data and generate shaped data; and
- generating means for generating based on the shaped data, in real time, a sound and/or tactile user signal, a frequency of the user signal depending on the shaped data.

9. A method for evaluation of a cosmetic effect of a cosmetic treatment comprising:

a) performing an evaluation of a condition of human hair with the method of any one of claims 1 to 7;
b) performing the cosmetic treatment; and
c) performing an evaluation of the same condition with the method of any one of claims 1 to 7.

**Patentansprüche**

1. Verfahren zum Informieren eines Benutzers über einen Zustand von menschlichem Haar, das aufweist:

a) über eine Messeinrichtung werden Messdaten über einen Evaluierungsbereich erhalten, wobei die Messeinrichtung konfiguriert ist, um Messdaten zu erzeugen, die sich auf den Haarzustand beziehen, wobei die Messeinrichtung gegenüber Reibung empfindlich ist und einen Koeffizienten der Reibung von Haar zur Verfügung stellt;

b) die Messdaten werden gestaltet, um gestaltete Daten zu erhalten, wobei die Gestaltung aufweist, Erzeugen von Daten basierend auf einem Vergleich von den Messdaten mit Bezugsdaten, die sich auf verschiedene Grade von Beschädigungen von Haaroberflächen beziehen; und **dadurch gekennzeichnet, dass**

c) in Echtzeit basierend auf den gestalteten Daten ein Ton-Benutzersignal und/oder ein taktiles Benutzersignal erzeugt wird, um durch den Benutzer wahrgenommen zu werden, wobei eine Frequenz von dem Benutzersignal von den gestalteten Daten abhängt.

2. Verfahren gemäß Anspruch 1, wobei das Gestalten das Filtern von den Messdaten aufweist.

3. Verfahren nach Anspruch 1 oder 2, wobei das Benutzersignal musikalische Noten aufweist.

4. Verfahren nach Anspruch 3, wobei das Benutzersignal eine Melodie aufweist.

5. Verfahren nach Anspruch 3 und/oder 4, wobei eine Variation einer Auf- und Abbewegung, einer Amplitude, einer Umhüllung, und eines Timbres basierend auf dem Ergebnis von einem Vergleich der gespeicherten Daten mit den Bezugsdaten erzeugt wird.

6. Verfahren nach Anspruch 1 oder 2, wobei das Verfahren aufweist, dass ein Spektrogramm basierend auf den gestalteten Daten erzeugt wird, und ein Geräusch bzw. Klang und/oder ein taktiles Benutzersignal basierend auf dem Spektrogramm erzeugt wird.

7. Verfahren nach irgendeinem der Ansprüche 1 bis 6, wobei der Benutzerklang bzw. das Benutzergeräusch oder das taktile Signal basierend auf einem kundenspezifischen Benutzerprofil erzeugt wird.

8. Einrichtung, die konfiguriert ist, um das Verfahren nach irgendeinem der Ansprüche 1 bis 7 durchzuführen, wobei die Einrichtung aufweist:

   - eine Akquisitionseinrichtung zum Erzeugen von Messdaten über menschliches Haar,
   - eine Verarbeitungseinrichtung zum Verarbeiten der Messdaten und zum Erzeugen von gestalteten Daten; und
   - eine Erzeugungseinrichtung zum Erzeugen eines Tones bzw. Geräusches und/oder taktilen Benutzersignals basierend auf gestalteten Daten in Echtzeit, wobei eine Frequenz von dem Benutzersignal von den gestalteten Daten abhängt.

9. Verfahren zum Bewerten von einem kosmetischen Effekt von einer kosmetischen Behandlung, das aufweist:

   a) Durchführung einer Bewertung von einem Zustand von menschlichem Haar mit dem Verfahren nach irgendeinem der Ansprüche 1 bis 7;
   b) Durchführen der kosmetischen Behandlung; und
   c) Durchführen einer Bewertung von dem gleichen Zustand mit dem Verfahren nach irgendeinem der Ansprüche 1 bis 7.

## Revendications

1. Procédé pour informer un utilisateur à propos d'un état de cheveux humains, comprenant :

   a) l'obtention par l'intermédiaire d'un dispositif de mesure de données de mesure à propos d'une zone en cours d'évaluation, dans lequel le dispositif de mesure est configuré pour générer des données de mesure relatives à l'état de cheveux, le dispositif de mesure étant sensible au frottement et fournissant un coefficient de frottement des cheveux ;
   b) la mise en forme des données de mesure pour obtenir des données mises en forme, la mise en forme comprenant la génération de données sur la base d'une comparaison des données de mesure avec des données de référence relatives à divers degrés de dommages à la surface des cheveux ; et **caractérisé par**
   c) la génération en temps réel, sur la base des données mises en forme, d'un signal utilisateur sonore et/ou d'un signal utilisateur tactile devant être perçu(s) par l'utilisateur, une fréquence du signal utilisateur dépendant desdites données mises en forme.

2. Procédé selon la revendication 1, dans lequel la mise en forme comprend le filtrage des données de mesure.

3. Procédé selon la revendication 1 ou 2, dans lequel le signal utilisateur comprend des notes de musique.

4. Procédé selon la revendication 3, dans lequel le signal utilisateur comprend une mélodie.

5. Procédé selon la revendication 3 et/ou 4, dans lequel une variation de hauteur, d'amplitude, d'enveloppe et de timbre est générée sur la base du résultat d'une comparaison des données mises en forme avec les données de référence.

6. Procédé selon la revendication 1 ou 2, le procédé comprenant la génération d'un spectrogramme sur la base des données mises en forme et la génération d'un signal utilisateur sonore et/ou tactile sur la base du spectrogramme.

7. Procédé selon l'une quelconque des revendications 1 à 6, dans lequel le signal sonore ou tactile utilisateur est généré sur la base d'un profil d'utilisateur personnalisé.

8. Dispositif configuré pour réaliser le procédé selon l'une quelconque des revendications 1 à 7, le dispositif comprenant :

   - des moyens d'acquisition pour générer des données de mesure à propos de cheveux humains ;
   - des moyens de traitement pour traiter les données de mesure et générer des données mises en forme ; et
   - des moyens de génération pour générer sur la base des données mises en forme, en temps réel, un signal utilisateur sonore et/ou tactile, une fréquence du signal utilisateur dépendant des données mises en forme.

9. Procédé d'évaluation d'un effet cosmétique d'un traitement cosmétique comprenant :

   a) la réalisation d'une évaluation d'un état de cheveux humains avec le procédé selon l'une quelconque des revendications 1 à 7 ;
   b) la réalisation du traitement cosmétique ; et
   c) la réalisation d'une évaluation du même état avec le procédé selon l'une quelconque des revendications 1 à 7 .

10 →

20 →

30 →

Fig. 1

Fig. 2

Fig. 3

Fig. 4

Fig. 5

Fig. 6

Fig. 7

Fig. 8A

Fig. 8B

Fig. 9A

Fig. 9B

Fig. 10A

Fig. 10B

Fig. 11A

Fig. 11B

14

Fig. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150201889 A
- JP 2012226472 A
- US 8440902 B
- US 2012041282 A1, NICHOL JAMIE GORDON [US]
- EP 1691195 A1
- JP 2004159830 A
- JP 2941114 B
- JP 2904616 B